# EUROPÄISCHE PATENTSCHRIFT

(11) **EP 0 780 357 B1**
(45) Veröffentlichungstag und Bekanntmachung des Hinweises auf die Patenterteilung: **18.10.2000**
(21) Anmeldenummer: 96119937.9
(22) Anmeldetag: 12.12.1996
(51) Int. Cl.: C07C 46/02, C07C 46/00, C07C 50/14

(54) **Herstellung von Vitamin K1**
Preparation of vitamin K1
Préparation de la vitamine K1

(30) Priorität: 20.12.1995 CH 359695
(43) Veröffentlichungstag der Anmeldung: 25.06.1997
(73) Patentinhaber: F. HOFFMANN-LA ROCHE AG, 4070 Basel (CH)
(72) Erfinder: Doerner, Manfred, Florence, South Carolina 29501 (US)
(74) Vertreter: Kellenberger, Marcus, Dr.

(56) Entgegenhaltungen:
- DE-B- 1 054 085
- US-A- 2 376 984
- US-A- 3 076 004
- CHEMICAL ABSTRACTS, vol. 080, no. 9, 4.März 1974 Columbus, Ohio, US; abstract no. 048207, MORITA E ET AL: "Quinone compounds" XP002025703 & JP 48 049 733 - (EISAI, CO., LTD.) 13.Juli 1973

## Beschreibung

Die vorliegende Erfindung betrifft ein Verfahren zur Herstellung von Vitamin K₁ durch Oxidation eines Dihydrovitamin K₁-Alkalimetallsalzes.

Gemäss dem einschlägigen Stand der Technik, über welchen in Houben-Weyl, Methoden der organischen Chemie, Bd. VII/13a, 1977, Seiten 39ff, berichtet wurde, lassen sich Hydrochinon, alkylsubstituierte Hydrochinone, 1,4-Dihydroxy-naphthalin sowie alkylsubstituierte Naphthaline sehr leicht zu den entsprechenden 1,4-Chinonen oxidieren, wobei nahezu alle bekannten Oxidationsmittel mehr oder weniger brauchbar sind. Im allgemeinen wird das Oxidationsmittel je nach Beständigkeit des herzustellenden Chinons ausgewählt, wobei für die Herstellung besonders empfindlicher Chinone Silber(I)-oxid in Diäthyläther oder Benzol in Gegenwart eines milden Trockenmittels, z.B. Natriumsulfat, das Reagens der Wahl ist. Weitere häufig verwendete Oxidationsmittel sind beispielsweise Eisen(III)-chlorid in Wasser oder wässrigem Aethanol, Natriumchlorat oder Kaliumbromat in verdünnter Schwefelsäure, Chrom(VI)-oxid in Schwefelsäure oder Essigsäure, Mangan(IV)-oxid in verdünnter Schwefelsäure, Kaliumnitrosodisulfonat und Thallium(III)-trifluoracetat. Gewisse dieser Oxidationsmethoden finden auch bei der Herstellung von Vitamin K₁ Verwendung und sind demzufolge von grosser praktischer Bedeutung; in der Vergangenheit hat man beispielsweise das Dihydrovitamin K₁-monoacetat verseift und anschliessend mit Silberoxid oder Eisenoxid zu Vitamin K₁ selbst oxidiert.

Die Ueberführung von unverestertem Dihydrovitamin K₁ in Vitamin K₁ mit Silberoxid ist insbesondere in den Arbeiten von L.F. Fieser (et al.) in J.A.C.S. 61 (1939), 2559 ff., 3467 und 3471, ibid. 62 (1940), 430 ff. und J. Biol. Chem. 133 (1940), 391 ff.; Isler et al., Helv. Chim. Acta 37 (1954), 225 und 230; Karrer et al., Helv. Chim. Acta 27 (1944), 317 ff.; Woods et al., Can. J. Chem. 35 (1957), 941 ff.; Mayer et al., Helv. Chim. Acta 47 (1964), 221 und 226; und Jackman et al., Helv. Chim. Acta 48 (1965), 1332 und 1346 beschrieben. Gemäss Jacob et al., J. Org. Chem. 41 (1976), 3627 ff., und neueren Berichten, z.B. Helv. Chim. Acta 73 (1990), 1276-1299 (s. Seiten 1280, 1283 und 1297) und CHIMIA 40 (1986), Nr. 9, 290-306 (s. Seiten 300-302) kann man Vitamin K₁ auch durch oxidative Demethylierung des Dimethylethers von Dihydrovitamin K₁ herstellen, und zwar unter Verwendung von Diammoniumcer(IV)hexanitrat als Katalysator in einem als Lösungsmittel fungierenden Gemisch von Benzol bzw. Essigsäure, Acetonitril und Wasser. Eine weitere Methode zur Herstellung von Vitamin K₁ besteht darin, Dihydrovitamin K₁-monoacetat oder -monobenzoat mit einem Alkalimetallhydroxid zu verseifen und anschliessend mit Luft zu oxidieren [Jackman et al., Helv. Chim. Acta 48 (1965), 1332; Schweizerische Patentschrift 320 582; und Isler et al., Helv. Chim. Acta 37 (1954), 225]. Anstelle eines Monoesters kann man auch einen Diester als Ausgangsmaterial verwenden, insbesondere das Diacetat, wie dies in der Japanischen Patentpublikation (Kokai) 212 142/1982 kurz erwähnt ist. Die Oxidation mit Luft wurde und wird immer noch als letzte Stufe vieler grossbetrieblicher Verfahren zur Herstellung von Vitamin K₁ verwendet, obwohl solche Verfahren u.a. den schwerwiegenden Nachteil aufweisen, dass auch das störende Nebenprodukt Vitamin K₁-Epoxid in beträchtlicher Menge entsteht.

Weitere Methoden zur Oxidation von Dihydrovitamin K₁ zu Vitamin K₁ und deren Nachteile sind in der Japanischen Patentpublikation (Kokai) 76854/1973 kurz angesprochen, und zwar die unwirtschaftlichen, umweltunfreundlichen und aufwendigen Oxidationen mit Silberoxid, Bleidioxid, Mangandioxid, Nickelperoxid und Dimethylsulfoxid. Die gegenständliche Erfindung dieser Patentpublikation besteht selber darin, Dihydrovitamin K₁ unter Verwendung von Wasserstoffperoxid (in wässriger Lösung) als Oxidationsmittel und gegebenenfalls in einem organischen Lösungsmittel, z.B. Hexan, Heptan, Diethylether, Diisopropylether, Petrolether, Petrolbenzol, Ligroin, Isopropanol, Dioxan, Benzol, Toluol usw., in Vitamin K₁ überzuführen.

Ziel der vorliegenden Erfindung ist es, ausgehend von dem bisher verwendeten Dihydrovitamin K₁-monoacetat-monobenzoat (nachfolgend "Dihydrovitamin K₁-acetatbenzoat") oder einem analogen Diester(-monoalkanoylat-monoaroylat) unter möglichst weitgehender Vermeidung der obenenwähnten Nachteile des Standes der Technik (insbesondere durch Reduktion der Lösungsmittel tragenden Abluft und Verringerung des Gehalts an Vitamin K₁-Epoxid) und in möglichst grosser Ausbeute Vitamin K₁ herzustellen. Dieses Ziel wird erfindungsgemäss erreicht, indem man nach Verseifung des Dihydrovitamin K₁-diesters mit einem Alkalimetallhydroxid zum entsprechenden Dihydrovitamin K₁-Alkalimetallsalz dieses nicht mit Luft sondern mit Wasserstoffperoxid in Gegenwart eines Eisen (III)-Salzes, insbesondere von Eisen (III)-sulfat oder Eisen (III)-chlorid, und bei einem pH-Wert im Bereich um etwa 14 zu Vitamin K₁ oxidiert.

Demgemäss handelt es sich bei dem erfindungsgemässen Verfahren um ein Verfahren (Oxidationsverfahren) zur Herstellung von Vitamin K₁ (I), das dadurch gekennzeichnet ist, dass man ein Alkalimetallsalz von Dihydrovitamin K₁ mit Wasserstoffperoxid in Gegenwart eines Eisen (III)-Salzes und bei einem pH-Wert von 13,7 bis 14,3 oxidiert. Der weitere Aspekt der vorliegenden Erfindung besteht darin, dass man zur Herstellung des im erfindungsgemässen Oxidationsverfahren verwendeten Dihydrovitamin K₁-Alkalimetallsalzes, insbesondere des Natrium- oder Kaliumsalzes, einen Dihydrovitamin K₁-diester der allgemeinen Formel 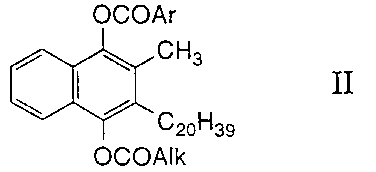 worin Ar eine Arylgruppe, Alk eine C₁₋₄-Alkylgruppe und
C₂₀H₃₉ die 3,7,11,15-Tetramethylhexadec-2-enylgruppe bedeuten,
mit Natrium- bzw. Kaliumhydroxid verseift.

In dieser Definition ist under dem Ausdruck "Arylgruppe" (Ar) im allgemeinen eine Phenyl- oder Naphthylgruppe zu verstehen, die jeweils beliebig substituiert sein kann, wobei die gegebenenfalls vorhandenen Substituenten insbesondere aus Niederalkylgruppen, Niederalkoxygruppen und Halogenatomen ausgewählt sind. Vorzugsweise bedeutet Ar jedoch unsubstituiertes Phenyl. Der Ausdruck "Niederalkylgruppe" bzw. Alk bezeichnet insbesondere eine C₁₋₄-Alkylgruppe, was sowohl für Alk als auch für die obenerwähnten Niederalkylgruppen als Substituenten gilt. Auch die obenerwähnte Niederalkoxygruppe enthält insbesondere 1 bis 4 Kohlenstoffatome. Unter Halogenatomen sind Fluor, Chlor, Brom und Jod zu verstehen. Vorzugsweise bedeutet Alk Methyl. Somit stellt die Formel II vorzugsweise Dihydrovitamin K₁-acetatbenzoat dar, wobei im Prinzip auch andere Diester der Formel II, wie diese anhand der obigen Erläuterung näher definiert sind, im erfindungsgemässen Verfahren verwendet werden können.

Zweckmässigerweise schliesst sich das erfindungsgemässe Oxidationsverfahren der Verseifung des Dihydrovitamin K₁-diesters an, ohne dass das Verseifungsprodukt (Dihydrovitamin K₁-Natriumsalz oder -Kaliumsalz) zuerst isoliert wird. Aus praktischen Gründen kann dieses zweistufige Verfahren folgendermassen durchgeführt werden:

Der Dihydrovitamin K₁-diester der Formel II wird mit einem Ueberschuss von Natrium- oder Kaliumhydroxid in einem zweiphasigen wässrig-organischen Lösungsmittelgemisch verseift. Dieses Lösungsmittelgemisch besteht zweckmässigerweise aus einer wässrigalkoholischen Phase und einer im wesentlichen mit dieser Phase nicht mischbaren oder darin nicht löslichen organischen Phase, wobei jede Phase gewünschtenfalls im voraus mit einem Aufhellungsmittel, z.B. einer wässrigen Lösung von Natriumdithionit, behandelt werden kann. Nach erfolgter Verseifung, die zweckmässigerweise unter Rühren bei Raumtemperatur oder nicht allsoviel erhöhter Temperatur bewerkstelligt werden kann, befindet sich das so hergestellte Dihydrovitamin K₁-Alkalimetallsalz grösstenteils in der wässrig-alkoholischen Phase. Diese Phase kann nach deren Abtrennung von der organischen Phase gewünschtenfalls mit frischem organischem Lösungsmittel (zweckmässigerweise desselben Types) gewaschen werden. Auch in diesem Fall kann das organische Lösungsmittel im voraus mit einem Aufhellungsmittel behandelt sein. Statt die organische Phase und Spülungen zu verwerfen, kann man aus wirtschaftlichen Gründen daraus das Lösungsmittel beispielsweise durch Destillation wiedergewinnen und recyclisieren. Dann wird zur abgetrennten, das Alkalimetallsalz enthaltenden wässrig-alkoholischen Lösung weiteres organisches Lösungsmittel gegeben, das gewünschtenfalls im voraus aufgehellt worden ist, und das resultierende zweiphasige Gemisch mit soviel Säure, die beliebig eine Mineralsäure oder eine organische Säure, wie beispielsweise wässrige Salzsäure oder Schwefelsäure bzw. Ameisensäure oder Essigsäure, vorzugsweise jedoch Essigsäure ist, gegeben, bis ein pH-Wert der wässrig-alkoholischen Phase von etwa 14 (im Bereich von 13,7 bis 14,3) erreicht worden ist. Man fügt anschliessend zum Ansatz eine wässrige Lösung des Eisen (III)-Salzes, gefolgt von einer wässrigen Lösung von Wasserstoffperoxid, hinzu, und zwar in beiden Fällen geeigneterweise unter Rühren und bei Raumtemperatur oder nicht allsoviel erhöhter Temperatur, beispielsweise bis etwa 40°C. Auf diese Weise erfolgt die durch das Eisen (III)-Salz katalysierte Oxidation des Dihydrovitamin K₁-Alkalimetallsalzes mit Wasserstoffperoxid zu Vitamin K₁. Das Produkt sammelt sich in der organischen Phase, und zwar in relativ kurzer Zeit, da die Oxidation normalerweise nur ein paar Minuten dauert. Das Ende der Oxidation wird in der Regel an einem Farbumschlag, etwa von braun nach gelb, leicht erkannt, und zudem an der Beobachtung, dass aus dem Reaktionsgemisch Sauerstoff kontinuierlich entweicht. Zur Aufarbeitung und Isolierung des Vitamins K₁ aus der organischen Phase wird diese zuerst abgetrennt. Sie kann dann mit Wasser, das gewünschtenfalls mit Essigsäure ergänzt ist, gewaschen und über einem Trockenmittel, z.B. wasserfreiem Natriumsulfat, getrocknet, eventuell mit einem Entfärbungsmittel, z.B. Aktivkohle oder Aluminiumoxid, behandelt und danach filtriert, und unter vermindertem Druck eingeengt werden. Der resultierende Niederschlag von Vitamin K₁ kann dann zweckmässigerweise durch Filtration aus dem eventuell im voraus mit Wasserdampf desodorierten Konzentrat isoliert werden. Das auf diese Weise hergestellte und isolierte Vitamin K₁ erweist sich als qualitativ sehr gut, da es sehr wenig Nebenprodukt Vitamin K₁-Epoxid enthält und normalerweise eine Reinheit von mehr als 98% aufweist. Auch die auf das Ausgangsmaterial Vitamin K₁-diester bezogene Ausbeute beträgt meistens mehr als etwa 85%.

Was das erfindungsgemässe Oxidationsverfahren betrifft, verwendet man zur Herstellung eines Gewichtsteils des Endproduktes Vitamin K₁ zweckmässigerweise 0.005 bis 0.01 Gewichtsteil des als Katalysator verwendeten Eisen (III)-Salzes [z.B. Fe₂(SO₄)₃; in der Praxis verwendet man allerdings in diesem Fall angesichts seiner leichten Erhältlichkeit vorzugsweise das Pentahydrat von Eisen (III)-sulfat, um die wässrige Lösung bereitszustellen, so dass die zweckmässigerweise verwendete Menge dieses Pentahydrats bezogen auf das Vitamin K₁ entsprechend höher ist]. Bezogen auf die in der Vorstufe (Verseifung) eingesetzte Menge Dihydrovitamin K₁-diester (z.B. -acetatbenzoat) verwendet man hingegen zweckmässigerweise 0,002 bis 0,01 Aequivalent Eisen (III)-Salz pro Aequivalent Diester, vorzugsweise 0,005 bis 0,007 Aequivalent Eisen (III)-Salz (im Falle des Acetatbenzoats und des Eisen (III)-sulfats entsprechen diese Bereiche 0,001 bis 0,01 Gewichtsteil Eisen (III)-sulfat pro Gewichtsteil Acetatbenzoat bzw. 0,003 bis 0,004 Gewichtsteil). Als wässrige Wasserstoffperoxidlösung wird zweckmässigerweise eine verwendet, welche eine Konzentration von 30 Gewichtsprozent (Gew.-%) bis 40 Gew.-% aufweist, vorzugsweise eine Konzentration von 35 Gew.-%. Bezogen auf die in der Verseifung eingesetzte Menge Dihydrovitamin K₁-diester verwendet man im Falle von Dihydrovitamin K₁-acetatbenzoat zweckmässigerweise 0,2 bis 0,6 Gewichtsteil, vorzugsweise 0,3 bis 0,35, wässrige Wasserstoffperoxidlösung, beispielsweise 35%-ige wässrige Wasserstoffperoxidlösung, pro Gewichtsteil Diester. Anders betrachtet, und zwar als Aequivalente ausgedrückt, verwendet man pro Aequivalent Diester zweckmässigerweise 1,5 bis 2,5 Aequivalente Wasserstoffperoxid als solches (H₂O₂), vorzugsweise 1,85 bis 2,13 Aequivalente. Basiert auf die Menge des schliesslich hergestellten Vitamins K₁ wird zweckmässigerweise pro Gewichtsteil dieses Produktes zweckmässigerweise 0,3 bis 0,8 Gewichtsteil wässrige Wasserstoffperoxidlösung (35%-ig, als Beispiel) verwendet, vorzugsweise 0,45 bis 0,53 Gewichtsteil. Wiederum anders betrachtet, verwendet man pro Aequivalent des schliesslich hergestellten Vitamins K₁ zweckmässigerweise 1,8 bis 2,8 Aequivalente Wasserstoffperoxid als solches (H₂O₂), vorzugsweise 2,1 bis 2,4 Aequivalente.

Wie oben erwähnt, erfolgt das erfindungsgemässe Oxidationsverfahren zweckmässigerweise in einem zweiphasigen wässrig-organischen Lösungsmittelgemisch, das einerseits aus einer wässrig-alkoholischen Phase und andererseits aus einer organischen Phase besteht. Als Alkohol eignet sich im allgemeinen ein C₁₋₃-Alkanol, d.h.Methanol, Ethanol, n-Propanol oder Isopropanol. Vorzugsweise wird jedoch Methanol verwendet. Bei der organischen Phase handelt es sich geeigneterweise um einen aliphatischer Kohlenwasserstoff, z.B. Hexan, Heptan oder ein Gemisch von Kohlenwasserstoffen, etwa Petrolether; einen halogenierten Kohlenwasserstoff, z.B. Methylenchlorid; einen aromatischen Kohlenwasserstoff, z.B. Benzol oder Toluol; oder Benzin oder eine Fraktion davon, z.B. Ligroin. Vorzugsweise wird als organische Phase Petrolether verwendet.

Das Volumenverhältnis Alkohol:Wasser in der wässrig-alkoholischen Phase beträgt zweckmässigerweise 3:1 bis 7:1. Im Falle der Verwendung des bevorzugten Alkohols Methanol beträgt dieses Volumenverhältnis im allgemeinen 3 : 1 bis 7 : 1, vorzugsweise 4 : 1 bis 6 : 1. Bezüglich des zweiphasigen Lösungsmittelgemisches beträgt das Volumenverhältnis wässrig-alkoholische Phase:organische Phase im allgemeinen 3 : 2 bis 2 : 3, vorzugsweise 10 : 8 bis 1 : 1. Man verwendet pro Gramm oder Aequivalent des zu oxidierenden Dihydrovitamin K₁-Alkalimetallsalzes (beispielsweise Kaliumsalz) zweckmassigerweise 0,001 bis 0,01 Liter bzw. 10 bis 25 Liter zweiphasiges Lösungsmittelgemisch, vorzugsweise 0,002 bis 0,006 Liter bzw. 15 bis 20 Liter.

Die Oxidation wird zweckmässigerweise bei Temperaturen im Bereich von etwa 15°C bis etwa 40°C, vorzugsweise bei der Raumtemperatur, durchgeführt.

Was die dem erfindungsgemässen Oxidationsverfahren vorgeschaltete Verseifung betrifft, verwendet man zweckmässigerweise pro Aequivalent Dihydrovitamin K₁-diester der Formel II etwa 22 bis 30 Aequivalente Natrium- bzw. Kaliumhydroxid, vorzugsweise 25 bis 27 Aequivalente dieser Base. Vorzugsweise wird Kaliumhydroxid als Base verwendet. Die Konzentration der verwendeten wässrigen Natrium- oder Kaliumhydroxidlösung beträgt zweckmässigerweise 40 Gewichtsprozent (Gew.-%) bis 60 Gew.-%, vorzugsweise 55 bis 58 Gew.-%. Auch die Verseifung erfolgt - wie oben erwähnt - zweckmässigerweise in einem zweiphasigen wässrig-organischen Lösungsmittelgemisch, das einerseits aus einer wässrig-alkoholischen Phase und andererseits aus einer organischen Phase besteht. Die oben angegebenen diesbezüglichen Angaben über die Natur und die relativen Volumenverhältnisse der Bestandteile dieses Lösungsmittelgemisches in bezug auf das Oxidationverfahren gelten auch im wesentlichen für die Verseifung. Bei der Verseifung verwendet man pro Gramm oder Aequivalent des zu verseifenden Dihydrovitamin K₁-diesters der Formel II (beispielsweise des Acetatbenzoats) zweckmässigerweise 0,01 bis 0,05 Liter bzw. 10 bis 25 Liter zweiphasiges Lösungsmittel-gemisch, vorzugsweise 0,02 bis 0,04 Liter bzw 15 bis 20 Liter. Die Verseifung wird zweckmässigerweise bei Temperaturen im Bereich von etwa 15°C bis etwa 30°C, vorzugsweise bei der Raumtemperatur, durchgeführt und dauert in der Regel eine bis drei Stunden, insbesondere etwa 2 Stunden.

Die sowohl bei der Verseifung als auch bei der anschliessenden Oxidation gegebenenfalls verwendete Aufhellung, z.B. mittels einer wässrigen Lösung von Natriumdithionit, dient dazu, das während der diesbezüglichen Reaktion zum Dunkeln tendierende Reaktionsgemisch heller und klarer zu machen, damit die jeweilige Umsetzung leichter optisch verfolgt werden kann. Zudem hat es sich erwiesen, dass durch Verwendung des Aufhellungsmittels das schliesslich erhaltene Vitamin K₁ eine bessere Qualität hat.

Die vorliegenden Erfindung, wird durch die nachfolgenden Beispiele veranschaulicht:

### Beispiel 1

In einem mit Thermometer, Kühler, Tropftrichter und Rührer ausgestatteten 1,5 l-Vierhalssulfierkolben werden unter Stickstoff 75 g (0,261 Mol) Kaliumhydroxid in 55,5 ml Wasser gelöst. Zur Lösung werden 329,5 ml Methanol zugegeben, wobei eine exotherme Reaktion erfolgt. Man kühlt die wässrig-methanolische Lösung innert 10 Minuten auf 10°C ab. Dann gibt man eine Lösung von 0,5 g Natriumdithionit in 7,5 ml Wasser zu und erwärmt das Gemisch in einem Wasserbad auf 20°C.

Anschliessend wird unter Rühren eine Lösung von 30 g (0,05 Mol) Dihydrovitamin K₁-acetatbenzoat in 550 ml im voraus mit 20 ml 10%iger wässriger Natriumdithionitlösung behandeltem, hochsiedendem Petrolether innert 5 Minuten zum obigen Gemisch bei 20-25°C zugetropft, und das Ganze wird 2 Stunden bei 20-24°C kräftig gerührt. Falls sich während des Rührens die Reaktionslösung braun verfärbt, wird sie rasch mit genügend 10%iger Natriumdithionitlösung aufgehellt. Zur Phasentrennung wird dann das Gemisch genügend lang stehen gelassen, und danach wird die obere Petroletherphase mittels eines Glasrohrs und Stickstoff abgedrückt und die untere methanolische Phase viermal mit jeweils 195 ml im voraus mit 10%iger wässriger Natriumdithionitlösung behandeltem, hochsiedendem Petrolether gewaschen. Während dieses Waschens soll die methanolische Lösung gelb sein, so dass eventuell nachdunkelnde Lösung sofort mit genügend 10%iger wässriger Natriumdithionitlösung aufgehellt wird. Die Petroletherextrakte werden verworfen. Auf diese Weise (Verseifung) erhält man eine gelbe methanolische Lösung von Dihydrovitamin K₁-Kaliumsalz.

Zur gelben Lösung der Vorstufe werden 500 ml im voraus mit 20 ml 10%iger wässriger Natriumdithionitlösung behandelter, hochsiedender Petrolether zugegeben. Unter kräftigem Rühren tropft man etwa 50-52 ml Eisessig, die letzten 5-7 ml besonders langsam, zu. Da die Reaktion exotherm ist, erfolgt diese Zugabe unter Eisbadkühlung bei 10°C derart, dass die Reaktionstemperatur 30°C nicht übersteigt. Zudem kann die Reaktionslösung beim Nachdunkeln durch Zugabe von 10%iger wässriger Natriumdithionitlösung wieder aufgehellt werden. Bei einem pH-Wert von 14 ist die Lösung grünstichig gelb, und die Zugabe von Eisessig wird dann beendet.

Man löst 175 mg Eisen(III)-sulfatpentahydrat in 7,5 ml Wasser und gibt unter Rühren die Lösung zum Reaktionsgemisch der Vorstufe zu, worauf sich das Gemisch olivgrün verfärbt (in dieser Reaktionsstufe wird keine Natriumdithionitlösung mehr zugegeben). Dann werden unter Rühren 7,9 ml wässrige Wasserstoffperoxidlösung innert 5 Minuten zugetropft, wobei die Temperatur der Reaktionslösung 30°C nicht übersteigen darf. Zu diesem Zweck wird die exotherme Reaktion durch Eisbadkühlung bei 5-10°C unter Kontrolle gehalten. (Es wird beobachtet, dass zu Beginn der Wasserstoffperoxidzugabe die Farbe der Reaktionslösung von olivgrün nach dunkelbraun umschlägt, und dass aus der Lösung Sauerstoff kontinuierlich entweicht. Im Verlaufe der Oxidation ändert sich die Farbe weiter von dunkelbraun über braun zu gelb; nach beendeter Oxidation ist sie goldgelb.)

Nach beendeter Reaktion wird die Lösung zur Phasentrennung genügend lang stehen gelassen. Dann trennt man die gelbe Petroletherphase ab und wäscht sie der Reihe nach mit 200 ml verdünnter Essigsäure (50 mg Eisessig in 200 ml Wasser), bis der pH-Wert der Essigsäurephase etwa 6-7 beträgt, und zweimal mit jeweils 200 ml Wasser. Die Petroletherphase wird über 19 g wasserfreiem Natriumsulfat getrocknet und anschliessend filtriert. Man gibt zur Lösung 0,75 g Aktivkohle und rührt das Gemisch 30 Minuten, danach 10 g alkalifreies, neutrales Aluminiumoxid enthaltend 2% Wasser (aus 9,8 g Aluminiumoxid und 0,2 g Wasser durch 1-stündiges Rühren bei Raumtemperatur hergestellt) mit weiterem 30-minütigem Rühren. Nach Abfiltration der Festkörper unter Stickstoff wird die Petroletherlösung bei 60°C/4.10³ Pa (40 mbar) ebenfalls unter Stickstoff eingeengt, und zwar in einem Kolben aus Braunglas, um das Gut auch vor Licht zu schützen. Schliesslich wird das so hergestellte gelbölige Vitamin K₁ unter Druck abfiltriert.

### Beispiel 2

Zu einer Lösung von etwa 30 kg Dihydrovitamin K₁-acetatbenzoat in etwa 550 l Petrolether unter Stickstoff wird bei Raumtemperatur eine Lösung von etwa 1,4 kg Natriumdithionit in etwa 10 l Wasser zugegeben. Es resultiert eine gelbe Lösung, die beim Nachdunkeln vor dem nächsten Schritt durch Zugabe weiterer Natriumdithionitlösung aufgehellt wird. Der Lösung gibt man anschliessend eine Lösung von etwa 75 kg Kaliumhydroxid in etwa 56 kg Wasser und etwa 253 kg Methanol zu, und das Gemisch wird etwa 2 Stunden gerührt.

Man trennt die methanolische Lösung ab und wäscht sie mehrmals mit Petrolether, wonach man die methanolische Lösung mit etwa 280 kg Petrolether und etwa 74 kg Methanol verdünnt und nach Bedarf durch Zugabe von wässriger Natriumdithionitlösung aufhellt. Unter Zugabe von 52-60 kg Essigsäure sowie nötigenfalls von weiterer Natriumdithionitlösung wird der pH-Wert auf 14 eingestellt. Zum Gemisch gibt man eine Lösung von etwa 0,15 kg Eisen(III)-sulfat in etwa 15 kg Wasser, gefolgt von 7-10 kg 35%iger wässriger Wasserstoffperoxidlösung. Die abgetrennte methanolische Phase wird mit Petrolether extrahiert, und die Petroletherphase wird zunächst mit sehr verdünnter wässriger Essigsäurelösung gewaschen und danach mit wasserfreiem Natriumsulfat getrocknet, das hinterher abgetrennt wird. Nach Entfärben der Petroletherphase mit etwa 1 kg Aktivkohle sowie etwa 10 kg Aluminiumoxid werden die Entfärbungsmittel durch Filtration abgetrennt und mit Petrolether gewaschen. Die vereinigten Petroletherfiltrate werden dann unter vermindertem Druck eingeengt. Man desodoriert den Rückstand mit Wasserdampf und filtriert den Niederschlag von Vitamin K₁ ab. Auf diese Weise erhält man eine 80-92%ige Ausbeute von Vitamin K₁.

### Beispiel 3

Zusammenfassung: Dihydrovitamin K₁-acetatbenzoat wird in Petrolether gelöst und die Lösung mit wässriger Natriumdithionitlösung entfärbt. Anschliessend wird mit einer wässrig-methanolischen Kalilauge 2 Stunden lang verseift und die methanolische Phase mit Petrolether extrahiert und nötigenfalls nochmals entfärbt. Nach Verdünnen der Petroletherphase mit weiterem Petrolether sowie Methanol wird durch Zugabe von Eisessig auf pH 14 gestellt. In Anwesenheit von Eisen(III)-sulfat als Katalysator wird dann mit Wasserstoffperoxid unterhalb von 30°C oxidiert. Man wäscht die Petroletherphase mit Wasser, trocknet sie mit wasserfreiem Natriumsulfat und entfärbt sie mit Aktivkohle mitsamt Aluminiumoxid. Das Filtrat wird unter vermindertem Druck eingeengt und mittels Wasserdampfdestillation gereinigt. Schliesslich wird das so erhaltene Vitamin K₁ abfiltriert.

### (i) Verseifung

Unter Stickstoff werden zu einem auf 20±1°C abgekühlten Gemisch von 56 l Wasser und 320 l (253 kg) Methanol 75 kg Kaliumhydroxid zugegeben, wobei die Temperatur auf 40-50°C ansteigt. Bei dieser Temperatur wird solange gerührt, bis das Kaliumhydroxid vollständig gelöst ist (45-60 Minuten), und dann wird die Lösung ["(a)"] auf 15-25°C abgekühlt.

In einem Rührkessel werden unter Stickstoff 30 kg rohes Dihydrovitamin K₁-acetatbenzoat in 500 l (335 kg) hochsiedendem Petrolether unter Rühren gelöst. Dann werden der Kesselinhalt und 50 l (35 kg) Petroletherspülungen [zusammen "Lösung (b)"] mit Stickstoff über Untenausläufe in einen weiteren Kessel eingeführt, in den hinterher auch die Lösung (a) eingeleitet wird. Zum Gemisch der Lösungen (a) und (b) wird zwecks Aufhellung auch noch eine Lösung von 1,4 kg Natriumdithionit in 10 l Wasser gegeben, und man rührt dann die ganze Verseifungslösung ["(c)"] 120-125 Minuten bei 20-25°C. Während der Rührdauer wird die Farbe der Verseifungslösung laufend kontrolliert: sollte die Farbe von gelb abweichen (dunkler werden), muss die Lösung durch Zugabe weiterer wässriger Natriumdithionitlösung (0,3 kg/5 l) aufgehellt werden. Nach Beendigung der Verseifung wird die Lösung (c) 20 bis höchstens 30 Minuten stehen gelassen.

### (ii) Extraktion vor Oxidation

In einem Rührkessel werden 640 l (429 kg) hochsiedender Petrolether mit einer Lösung von 0,3 kg Natriumdithionit in 5 l Wasser behandelt. Nach Abtrennung der wässrigen Phase verbleibt die Petroletherphase ["(d)"].

Zur 160 l (107 kg) der Petroletherphase (d) in einem Rührkessel wird die untere methanolische Phase der Lösung (c) zwecks Extraktion gegeben, und nach Abtrennung der Petroletherphase wird die untere methanolische Phase nochmals mit 160 l (107 kg) der Petroletherphase (d) extrahiert. Diese zweifache Extraktion der methanolischen Phase wird noch einmal wiederholt. Es bleibt nach Abtrennung der Petroletherphase die methanolische Phase [(e)].

In einem Rührkessel werden 560 l (429 kg) hochsiedender Petrolether mit einer Lösung aus 0,3 kg Natriumdithionit in 5 l Wasser behandelt. Nach Abtrennung der wässrigen Phase verbleibt die Petroletherphase [(f)].

Zur 100 l (67 kg) der Petroletherphase (f) in einem Rührkessel wird die methanolische Phase (e) zwecks nochmaliger Extraktion gegeben, und nach Abtrennung der Petroletherphase verbleibt die methanolische Phase [(g)], welche aus einer methanolischen Lösung von Dihydrovitamin K₁-Kaliumsalz besteht.

### (iii) Vorbereitung zur Oxidation

Zu 10 l Wasser wird unter Rühren 0,5 kg Natriumdithionit zugegeben, gefolgt von weiteren 3 l Wasser, und das Ganze wird 5 Minuten nachgerührt [Lösung (h)].

In einem separaten Kessel wird unter Rühren 0,15 kg Eisen(III)-sulfathydrat zu 7 l Wasser zugegeben, gefolgt von weiteren 3 l Wasser, und das Ganze wird 5 Minuten nachgerührt (Eisensulfatlösung).

200 l (134 kg) hochsiedender Petrolether werden in einen Oxidationskessel eingeführt, und zu diesem Petrolether wird die methanolische Phase (g) gegeben. Es folgt die Zugabe eines Gemisches aus 174 kg (260 l) hochsiedendem Petrolether und 74 kg (94 l) Methanol. Ist der Inhalt des Oxidationskessels nicht zitronengelb, werden noch 1-5 l (1,1-5,4 kg) wässrige Natriumdithionitlösung solange unter Rühren zugegeben, bis die Farbe von braun nach zitronengelb umschlägt.

Bei einer Temperatur unterhalb von 30°C werden 53-57 l (52-60 kg) Eisessig zur Lösung im Oxidationskessel langsam zudosiert, bis ein pH-Wert von 14 erreicht worden ist. Falls die Lösung nicht mehr zitronengelb ist, werden erneut 0,1-5 l (0,1-5,4 kg) wässrige Natriumdithionitlösung langsam zugegeben.

Zur Lösung im Oxidationskessel wird dann die Eisensulfatlösung zugegeben, gefolgt von 5 l Wasserspülungen.

### (iv) Oxidation

Zum Gemisch im Oxidationskessel werden innerhalb von 4-6 Minuten bei 27-30°C 7-8,5 l (7,7-9,6 kg) 35%ige wässrige Wasserstoffperoxidlösung zudosiert. Im Verlauf der resultierenden Oxidation schlägt die Farbe zunächst von olivgrün nach dunkelbraun um; danach hellt sich der Ansatz auf, wird kurz dunkler und schlägt dann sofort nach gelb um. Sobald der Ansatz gelb gefärbt ist, wird die Wasserstoffperoxidzugabe unterbrochen.

### (v) Extraktion nach Oxidation

Nach erfolgter Oxidation und Phasentrennung wird die untere Methanolphase in einen Rührkessel übertragen, in dem sich bereits 150 l (100 kg) Petrolether befinden. Nach Extraktion wird die weitere methanolische Phase entfernt. Es verbleibt die Petroletherphase [(i)].

Die noch im Oxidationskessel befindliche Petroletherphase wird mit 200 l Wasser gereinigt und nach Zugabe von 0,5-0,8 l (0,52-0,84 kg) Eisessig bei pH 3,6-4,0 extrahiert. Dann wird die abgetrennte Petroletherphase mitsamt Spülungen mit 100 l (67 kg) hochsiedendem Petrolether in einen Spitzkessel übertragen und dort mit 200 l Wasser gereinigt. Nach Abtrennung der unteren wässrigen Phase wird diese in einem weiteren Kessel mit 100 l (67 kg) hochsiedendem Petrolether extrahiert und deren pH-Wert gemessen. Sollte er grösser als 6 betragen, wird die im Spitzkessel befindliche Petroletherphase erneut mit 200 l Wasser gereinigt und die untere wässrige Phase abgetrennt und deren pH-Wert gemessen. Man wiederholt diese Prozedur, bis der gemessene pH-Wert der wässrigen Phase weniger als 6 beträgt, und zwar eventuell viermal. Die auf diese Weise gereinigte Petroletherphase besteht fast ausschliesslich aus Vitamin K₁ und Lösungsmittel.

### (vi) Aufarbeitung der Petroletherphase

30 l der im Spitzkessel befindlichen Petroletherlösung wird in einen weiteren Kessel übertragen, die restliche (der Grossteil der) Petroletherlösung in einen Rührkessel übertragen, und die 30 l Petroletherlösung wieder in den Spitzkessel zurückgeführt, um dort mit einer aus 100 l Wasser und 50 ml (0,053 kg) Eisessig bestehenden Essigsäurelösung extrahiert zu werden. Die untere wässrige Phase dieser Extraktion wird abgetrennt und deren pH-Wert (Sollwert: 4-5) gemessen. Dann extrahiert man die Petroletherlösung im Spitzkessel mit 100 l Wasser und misst den pH-Wert (Sollwert: 6-7) der abgetrennten unteren wässrigen Phase. Schliesslich wird die im Spitzkessel verbleibende Petroletherlösung mit derjenigen (dem Grossteil, (i)) im Rührkessel vereinigt.

### (vii) Trocknung und Entfärbung

Zur Petroletherlösung im Rührkessel gibt man 10 kg wasserfreies Natriumsulfat. Nach 30-minütigem Rühren und Trocknen wird das Trockenmittel durch Filtration abgetrennt und die filtrierte Petroletherlösung mitsamt ebenfalls filtrierten Petroletherspülungen (50 l) aus dem Rührkessel wieder in den Rührkessel übertragen. Unter Stickstoff wird zur Petroletherlösung 1,0 kg Aktivkohle zugegeben, und das Gemisch wird 30 Minuten gerührt. Nach Zusatz von 10 kg durch 200 ml Wasser desaktiviertem, alkalifreiem Aluminiumoxid wird erneut 30 Minuten gerührt. Dann werden die Suspension und zwei Petroletherspülungen (jeweils 100 l) filtriert.

Die Entfärbung mit Aktivkohle und Aluminiumoxid wird wiederholt und ein Muster des Filtrats auf Farbe und Fluoreszenz geprüft. Je nach Resultat wird der Vorgang mit Aktivkohle allein (zur Entfärbung) oder mit Aluminiumoxid allein (zur Entfernung floreszierender Anteile) wiederholt, bis die Petroletherlösung entfärbt ist.

### (viii) Eindampfen der das Vitamin K₁ enthaltenden Petroletherlösung

Unter einem Druck von 20 kPa (200 mbar) wird die entfärbte Petroletherlösung bei 85-90°C (Dampfaustritt) und einer Durchlaufgeschwindigkeit von 120-130 l/h unter Rühren kontinuierlich eingedampft. Man überträgt dann die aufkonzentrierte Vitamin K₁-Lösung in einen Kessel und überlagert sie unter Stickstoff bei einem Druck von 30 kPa (0,3 bar).

Das Eindampfen und anschliessende Ueberlagerung (unter einem Stickstoffdruck von 30 kPa (0,3 bar) der aufkonzentrierten Vitamin K₁-Lösung werden zweimal wiederholt (1. Wiederholung: 3-4 kPa (30-40 mbar), Dampfaustritt 70-80°C, Durchlaufgeschwindigkeit 30-40 l/h; 2. Wiederholung: <2.5 kPa (25 mbar), Dampfaustritt 55-65°C, Durchlaufgeschwindigkeit 80-110 l/h).

### (ix) Desodorierung mit Wasserdampf

Der Inhalt des letzten Ueberlagerungskessels wird bei weniger als 2.5 kPa (25 mbar) und einer Manteltemperatur von 68-74°C einer Wasserdampfdestillation unterzogen. Anschliessend wird das aufgefangene Vitamin K₁ unter Stickstoff bei einem Druck von 30 kPa (0,3 bar) überlagert.

### (x) Feinfiltration und Abfüllung

Drei Ansätze Vitamin K₁ werden mittels einer Zahnradpumpe bei einem Druck von höchstens 4 bar durch eine Kombination von drei Filtern (2/10/0,2 µm) filtriert, aufgefangen und bemustert. Ist die Analyse zufriedenstellend, wird mit zwei weiteren Ansätzen in gleicher Weise verfahren.

Nach Mischen dreier Ansätze wird das Gemisch analysiert, um die hohe Qualität zu bestätigen.

Nach einstündigem Rühren unter Stickstoff kann das so erhaltene Vitamin K₁ guter Qualität (Reinheit) in Bidons abgefüllt und mit Stickstoff überlagert werden. Anschliessend werden die Bidons verschlossen und etikettiert.

## Patentansprüche

1. Verfahren zur Herstellung von Vitamin K₁, dadurch gekennzeichnet, dass man ein Alkalimetallsalz von Dihydrovitamin K₁ mit Wasserstoffperoxid in Gegenwart eines Eisen (III)-Salzes und bei einem pH-Wert von 13,7 bis 14,3 oxidiert.

2. Verfahren nach Anspruch 1, dadurch gekennzeichnet, dass man zur Herstellung des Dihydrovitamin K₁-Alkalimetallsalzes, insbesondere des Natrium- oder Kaliumsalzes, einen Dihydrovitamin K₁-diester der allgemeinen Formel 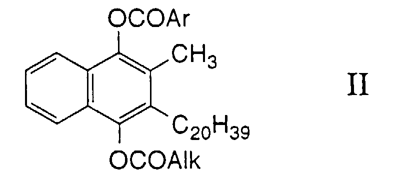 worin Ar eine Arylgruppe, Alk eine C₁₋₄-Alkylgruppe und
C₂₀H₃₉ die 3,7,11,15-Tetramethylhexadec-2-enylgruppe bedeuten, mit Natrium- bzw. Kaliumhydroxid verseift.

3. Verfahren nach Anspruch 1 und 2, dadurch gekennzeichnet, dass sich das Verfahren nach Anspruch 1 dem Verfahren nach Anspruch 2 anschliesst, ohne dass das Verseifungsprodukt Dihydrovitamin K₁-Natriumsalz oder -Kaliumsalz zuerst isoliert wird.

4. Verfahren nach Anspruch 2 oder 3, dadurch gekennzeichnet, dass als Dihydrovitamin K₁-diester das Acetatbenzoat verwendet wird.

5. Verfahren nach einem der Ansprüche 1 bis 4, dadurch gekennzeichnet, dass das jeweilige Verfahren in einem zweiphasigen wässrig-organischen Lösungsmittelgemisch erfolgt.

6. Verfahren nach Anspruch 5, dadurch gekennzeichnet, dass das wässrig-organische Lösungsmittelgemisch aus einer wässrig-alkoholischen Phase und einer im wesentlichen mit dieser Phase nicht mischbaren oder darin nicht löslichen organischen Phase besteht.

7. Verfahren nach Anspruch 6, dadurch gekennzeichnet, dass die wässrig-alkoholische Phase wässriges Methanol und die organische Phase Petrolether ist.

8. Verfahren nach einem der Ansprüche 1 bis 7, dadurch gekennzeichnet, dass als Eisen (III)-Salz Eisen (III)-sulfat verwendet wird.

9. Verfahren nach einem der Ansprüche 1 bis 8, dadurch gekennzeichnet, dass der pH-Wert, bei dem das Alkalimetallsatz von Dihydrovitamin K₁ zu Vitamin K₁ oxidiert wird, durch Zugabe von Essigsäure erreicht wird.

## Claims

1. A process for the manufacture of vitamin K₁, which process comprises oxidizing an alkali metal salt of dihydrovitamin K₁ with hydrogen peroxide in the presence of an iron(III) salt and at a pH of 13.7 to 14.3.

2. A process according to claim 1, wherein the dihydrovitamin K₁ alkali metal salt, especially the sodium or potassium salt, is produced by saponifying a dihydrovitamin K₁ diester of the general formula 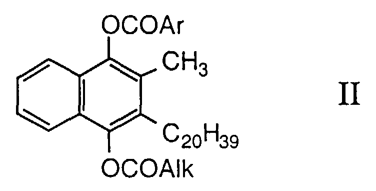 wherein Ar signifies an aryl group, Alk signifies a C₁₋₄-alkyl group and
C₂₀H₃₉ signifies the 3,7,11,15-tetramethylhexadec-2-enyl group, with sodium or potassium hydroxide.

3. A process according to claim 1 and 2, wherein the process according to claim 1 follows the process according to claim 2 without firstly isolating the dihydrovitamin K₁ sodium salt or potassium salt obtained as the saponification product.

4. A process according to claim 2 or claim 3, wherein the acetate benzoate is used as the dihydrovitamin K₁ diester.

5. A process according to any one of claims 1 to 4, wherein the respective process is carried out in a two-phase aqueous-organic solvent mixture.

6. A process according to claim 5, wherein the aqueous-organic solvent mixture consists of an aqueous-alcoholic phase and an organic phase which is not significantly miscible with this phase or not significantly soluble therein.

7. A process according to claim 6, wherein the aqueous-alcoholic phase is aqueous methanol and the organic phase is petroleum ether.

8. A process according to any one of claims 1 to 7, wherein iron(III) sulphate is used as the iron(III) salt.

9. A process according to any one of claims 1 to 8, wherein the pH at which the dihydrovitamin K₁ alkali metal salt is oxidized to vitamin K₁ is achieved by the addition of acetic acid.

## Revendications

1. Procédé de préparation de vitamine K₁ , caractérisé en ce qu'on oxyde un sel de métal alcalin de dihydrovitamine K₁ avec du peroxyde d'hydrogène en présence d'un sel de fer(III) et à un pH de 13,7 à 14,3.

2. Procédé selon la revendication 1, caractérisé en ce que pour préparer le sel de métal alcalin de dihydrovitamine K₁, en particulier le sel de sodium ou de potassium, on saponifie avec de l'hydroxyde de sodium ou selon les cas de potassium un diester de dihydrovitamine K₁ de formule générale 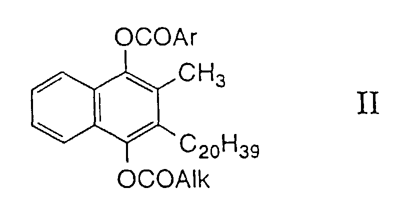 dans laquelle Ar représente un groupe aryle, alk représente un groupe alkyle en C₁ à C₄ et C₂₀H₃₉ représente le groupe 3,7,11,15-tétraméthylhexadéc-2-ényle.

3. Procédé selon les revendications 1 et 2, caractérisé en ce que le procédé selon la revendication 1 s'ajoute au procédé selon la revendication 2, sans qu'on n'isole tout d'abord le produit de saponification sel de sodium ou de potassium de dihydrovitamine K₁.

4. Procédé selon la revendication 2 ou 3, caractérisé en ce qu'on utilise comme diester de dihydrovitamine K₁ l'acétate-benzoate.

5. Procédé selon l'une des revendications 1 à 4, caractérisé en ce que le procédé en question se déroule dans un mélange de solvants aqueux-organique à deux phases.

6. Procédé selon la revendication 5, caractérisé en ce que le mélange de solvants aqueux-organique se compose d'une phase aqueuse-alcoolique et d'une phase organique essentiellement non miscible avec cette phase ou qui n'y est pas soluble.

7. Procédé selon la revendication 6, caractérisé en ce que la phase aqueuse-organique est le méthanol aqueux et la phase organique l'éther de pétrole.

8. Procédé selon l'une des revendications 1 à 7, caractérisé en ce qu'on utilise comme sel de fer (III) le sulfate de fer (III).

9. Procédé selon l'une des revendications 1 à 8, caractérisé en ce qu'on atteint le pH auquel le sel de métal alcalin de dihydrovitamine K₁ est oxydé en vitamine K₁ par addition d'acide acétique.
